# EUROPÄISCHE PATENTSCHRIFT

(11) **EP 3 409 207 B1**
(45) Veröffentlichungstag und Bekanntmachung des Hinweises auf die Patenterteilung: **31.08.2022**
(21) Anmeldenummer: 17000943.5
(22) Anmeldetag: 01.06.2017
(51) Int. Cl.: A61B 6/10, G21F 3/03, G21F 1/12

(54) **VORRICHTUNG ZUM STRAHLENSCHUTZ UND VERFAHREN ZU DEREN HERSTELLUNG**
DEVICE FOR RADIATION PROTECTION AND METHOD FOR THEIR PREPARATION
DISPOSITIF DE PROTECTION CONTRE LES RAYONNEMENTS ET SON PROCÉDÉ DE FABRICATION

(43) Veröffentlichungstag der Anmeldung: 05.12.2018
(73) Patentinhaber: MD Solutions GmbH, 61184 Karben (DE)
(72) Erfinder: Ernst, Philipp, 61184 Karben (DE)
(74) Vertreter: Bosch Jehle Patentanwaltsgesellschaft mbH

(56) Entgegenhaltungen:
- WO-A1-2016/090651
- CN-U- 205 656 862
- DE-B3-102014 215 448
- JP-U- 3 180 634
- US-A- 3 093 829
- US-A1- 2003 010 939
- US-A1- 2013 112 898
- US-A1- 2013 325 357
- US-A1- 2014 275 939

## Beschreibung

Die vorliegende Erfindung bezieht sich auf eine Vorrichtung zur Verwendung als Schutz vor gesundheitsschädlichen Strahlen. Die Erfindung bezieht sich ferner auf Verfahren zur Herstellung einer Vorrichtung zur Verwendung als Schutz vor schädlichen Strahlen.

Schutzvorrichtungen vor schädlichen Strahlen sind in zahlreichen Bereichen gebräuchlich und können in unterschiedlichen Größen und Formen ausgestaltet sein. So können beispielsweise ortsfeste Schutzeinrichtungen fest installiert sein, um ganze Räume vor schädlichen Strahlen abzuschirmen. Aus der DE 10 2011 122 745 A1 ist eine Strahlenschutzwand zur Abschirmung von Räumen bekannt, die eine Materialkombination zum Schutz vor radioaktiver Strahlung, insbesondere Röntgen- und Gammastrahlung enthält.

Die erfindungsgemäße Vorrichtung kann insbesondere beim Strahlenschutz von Ärzte- oder Behandlungspersonal eingesetzt werden, die sich in der Nähe von elektromagnetischen Strahlenquellen aufhalten, wie sie beispielsweise bei der Anwendung von Röntgenstrahlen zum Einsatz kommen. Bei der Untersuchung eines Patienten an einem Untersuchungstisch besteht die Gefahr einer Strahlenexposition des Untersuchers und Hilfspersonals.

Zur Verminderung der Strahlenexposition sind verschiedene Arten von Röntgenschutzkleidung bekannt. So sind beispielsweise für den medizinischen Bereich bei Untersuchungen und Behandlungen unter Anwendung von Röntgenstrahlen Strahlenschutzschürzen gebräuchlich, die mit Bleiglätte oder einem anderen Strahlenschutzstoff gefüllt sind. In der DE1897070 ist eine Strahlenschutzschürze mit Bleieinlage beschrieben, die dem Chirurgen bei der Anwendung von Röntgen-Durchleuchtungseinrichtungen Schutz bietet, ohne den Arzt bei seiner Tätigkeit zu behindern.

Aus der US 2013/112898 A1 ist eine weitere Strahlenschutzschürze bekannt, die mittels gezielter Zufuhr einer Röntgenabschirmflüssigkeit eine dynamisch veränderliche Schutzwirkung erreicht. Mindestens eine Röntgenstrahlungssensorvorrichtung ist auf der Innenseite der Strahlenschutzschürze angeordnet, um die Schutzwirkung zu überprüfen.

Trotz oder gerade bei Verwendung solcher Strahlenschutzvorrichtungen besteht das Bedürfnis, festzustellen, ob und inwieweit die Personen oder der Raum, der durch die Strahlenschutzvorrichtung abzuschirmen war, tatsächlich vor den schädlichen Strahlen geschützt waren, also ob die Strahlenschutzvorrichtung ihren Zweck erfüllt hat. Bislang wird diese Frage dadurch beantwortet, dass die zu schützenden Personen ein Dosimeter am Körper tragen, das die Dosis einer einfallenden Strahlung misst.

Ein Problem bei der Verwendung solcher am Körper getragenen Dosimeter besteht darin, dass die Personen sie zuweilen unbeabsichtigt nicht anlegen. Im Falle einer stationär eingerichteten Schutzvorrichtung besteht das Problem darin, dass eine in dem zu schützenden Raum eventuell anfallende Strahlenbelastung nicht gemessen werden kann, wenn sich dort kein Dosimeter oder Messgerät befindet.

Ein weiteres Problem besteht darin, dass tragbare Dosimeter weder ortsfest noch personengebunden sind und deshalb keine zuverlässigen Daten zur Strahlenbelastung eines bestimmten Ortes oder einer bestimmten Person liefern. Die vorliegende Erfindung hat zur Aufgabe, eine Strahlenschutzvorrichtung zu schaffen, welche die oben genannten Probleme beseitigt oder zumindest minimiert.

Die vorliegende Erfindung löst die oben genannte Aufgabe durch eine Vorrichtung zum Schutz vor schädlicher Strahlung, die eine Schutzschicht zum Schutz der schädlichen Strahlung umfasst, wobei in der Schutzvorrichtung mindestens eine Messvorrichtung zum Detektieren der schädlichen Strahlung integriert ist.

Die Messvorrichtung ist auf einer der Einfallsrichtung der Strahlung abgewandten Seite der Schutzschicht angeordnet. Die Messvorrichtung ist zudem mit einem RFID-Chip gekoppelt oder selbst als RFID Chip ausgebildet, der einen Speicher zum Speichern von Daten zur detektierten Strahlung sowie eine Schnittstelle zur drahtlosen Kommunikation mit einer äußeren Sende- und Empfangseinheit umfasst.

Nach einem weiteren Aspekt der vorliegenden Erfindung werden die Aufgaben gelöst durch ein Verfahren zur Herstellung einer Vorrichtung zum Schutz vor schädlicher Strahlung mit den im unabhängigen Verfahrensanspruch definierten Merkmalen.

Bisher bekannte Strahlenschutzvorrichtungen erfüllen lediglich den Zweck, schädliche Strahlen abzuschirmen und umfassen daher keinerlei Messvorrichtung, mit der eine schädliche Strahlung detektiert werden könnte. Sofern neben dem Strahlenschutz auch die Dosis der schädlichen Strahlen festgestellt werden soll, erfolgt dies bislang ausschließlich über separate Dosimeter, die von der Strahlenschutzvorrichtung unabhängig sind.

Die vorliegende Erfindung kombiniert die Strahlenschutzvorrichtung mit einer Messvorrichtung zum Detektieren der schädlichen Strahlung, indem sie die Messvorrichtung in der Strahlenschutzvorrichtung integriert. Daraus ergibt sich der Vorteil, dass die Messvorrichtung stets mit der Schutzvorrichtung fest und unverlierbar verbunden ist. Auf diese Weise kann sichergestellt werden, dass die in der Strahlenschutzvorrichtung integrierte Messvorrichtung zum einen nur die Strahlung detektiert, die an der Strahlenschutzvorrichtung auftrifft und zum anderen sämtliche Strahlung detektiert, die an der Strahlenschutzvorrichtung angefallen ist.

Durch die in der Strahlenschutzvorrichtung integrierte Messvorrichtung kann auch ein unbeabsichtigtes Vergessen der Messvorrichtung verhindert werden. Ein weiterer Vorteil der in der Strahlenschutzvorrichtung integrierten Messvorrichtung besteht darin, dass zuverlässig die an einer Strahlenschutzvorrichtung anfallende Strahlung erfasst wird, ohne dass der Benutzer der Schutzvorrichtung an die Messvorrichtung denken oder diese aktivieren muss. Dies ist insbesondere dann von Vorteil, wenn es sich um eine stationär eingerichtete Schutzvorrichtung handelt und sich in dem zu schützenden Bereich kein Dosimeter oder Messvorrichtung befindet, um die dort anfallende Strahlenbelastung zu messen.

Die erfindungsgemäße Vorrichtung zeichnet sich ferner dadurch aus, dass durch die Integration der Messvorrichtung in einer Strahlenschutzschürze ein angenehmerer Tragekomfort sowie ein ansprechender optischer Eindruck erreicht wird, als wenn die Messvorrichtung von außen an der Strahlenschutzschürze befestigt werden müsste und dadurch davon abstehen würde und evtl. in sterilen Bereichen zu Kontaminationen führen würde.

Gemäß der vorliegenden Erfindung ist die in der Strahlenschutzvorrichtung integrierte Messvorrichtung auf einer der Einfallsrichtung der Strahlung abgewandten Seite der Schutzschicht angeordnet. Mit dieser Anordnung kann die auf der Seite der Schutzschicht anfallende Strahlung gemessen werden, die von der Einfallsrichtung der Strahlung abgewandt ist. Im Falle einer Strahlenschutzschürze wäre dies die Rückseite, die am Körper des Trägers anliegt. Das heißt, dass damit die Strahlung gemessen würde, welche die Schutzschicht durchdringt oder die Strahlenschutzvorrichtung aus einer von der Haupteinfallsrichtung der Strahlung abweichenden Richtung erreicht. Dies kann beispielsweise durch Streustrahlung erfolgen, die den Körper des Trägers einer Strahlenschutzschürze an ungeschützter Stelle erreicht und durchdrungen hat.

Zudem kann eine weitere in der Strahlenschutzvorrichtung integrierte Messvorrichtung auf einer der Einfallsrichtung der Strahlung zugewandten Seite der Schutzschicht angeordnet sein.

Mit dieser Anordnung kann die auf der Seite der Schutzschicht anfallende Strahlung gemessen werden, die von der Einfallsrichtung der Strahlung zugewandt ist. Im Falle einer Strahlenschutzschürze wäre dies die Vorderseite, die gegen die Strahlungsquelle gerichtet ist. Das heißt, dass damit die Strahlung gemessen würde, die von der Strahlungsquelle auf die Schutzschicht trifft.

Somit ist dann eine in der Strahlenschutzvorrichtung integrierte Messvorrichtung auf einer der Einfallsrichtung der Strahlung abgewandten Seite der Schutzschicht angeordnet , und eine weitere in der Strahlenschutzvorrichtung integrierte Messvorrichtung auf einer der Einfallsrichtung der Strahlung zugewandten Seite der Schutzschicht angeordnet.

Dadurch kann gleichzeitig sowohl die auf der einen Seite der Schutzschicht anfallende Strahlung als auch die auf der anderen Seite der Schutzschicht ankommende Strahlung bzw. eine die Schutzschicht durchdringende Strahlung gemessen werden.

Eine Strahlenschutzvorrichtungkann eine Aufzeichnungsvorrichtung zum Aufzeichnen der von der Messvorrichtung erfassten Dosis der Strahlung in der Schutzvorrichtung umfassen.

Diese Aufzeichnungsvorrichtung ist wie die Messvorrichtung vorzugsweise ebenfalls in der Strahlenschutzvorrichtung integriert, d.h. im Material oder Schichtaufbau der Strahlenschutzvorrichtung enthalten. Ferner ist die Aufzeichnungsvorrichtung mit der Messvorrichtung derart gekoppelt, dass sie die von der Messvorrichtung detektierte Strahlung bzw. die von der Messvorrichtung erfasste Strahlungsdosis aufzeichnen kann.

Dazu umfasst die Aufzeichnungsvorrichtung beispielsweise ein Speichermedium, in dem Daten zur erfassten Strahlung bzw. zur erfassten Strahlungsdosis gespeichert werden können. Mittels des Speichermediums der Aufzeichnungsvorrichtung können die Daten zur erfassten Strahlung beispielsweise in zeitlicher Abfolge, inkrementell, als Summe über einzelne Zeitabschnitte oder als Summe über die gesamte Einsatzdauer der Strahlenschutzvorrichtung gespeichert werden.

Die Messvorrichtung kann mit der

Aufzeichnungsvorrichtung in einer Mess- und Aufzeichnungsvorrichtung kombiniert sein. Ferner können Mittel zur Erfassung der räumlichen Position der Messvorrichtung oder der Mess- und Aufzeichnungsvorrichtung vorgesehen sein. Die ermittelten Daten zur räumlichen Position der Messvorrichtung können ebenfalls in der Aufzeichnungsvorrichtung gespeichert werden. Auf diese Weise können die Daten zur räumlichen Position mit den Daten zur erfassten Strahlung bzw. zur erfassten Strahlungsdosis miteinander in Bezug gebracht werden. Aufgrund solcher Daten können dann Histogramme oder räumliche Auswertungen zur Strahlungsbelastung erzeugt werden.

Mithin können die Daten zur erfassten Strahlung Informationen zur Dosis, Zeitpunkt, Zeitspanne, Intensität, Strahlungsart, Strahlungsrate, Gesamtmenge der erfassten Strahlung, räumlichen Position der Mess- und Aufzeichnungsvorrichtung und/oder dem zeitlichen Verlauf der genannten Observablen bzw. Beobachtungswerte umfassen.

Zum Auslesen der in der Aufzeichnungsvorrichtung gespeicherten Daten sind vorzugsweise Mittel zur drahtgebundenen oder drahtlosen Kommunikation mit einer Sende- und Empfangseinheit, einer Datenverarbeitungseinheit und/oder einer Bilderzeugungseinheit vorgesehen. Auf diese Weise kann eine Schnittstelle eingerichtet werden, durch die die in der Aufzeichnungsvorrichtung gespeicherten Daten ausgelesen und einer weiteren Datenverarbeitung zugänglich gemacht werden können. Die Schnittstelle ist vorzugsweise mit einer solchen Bandbreite ausgestattet, die für eine verzögerungsfreie Übertragung der Daten zur erfassten Strahlung ausreichend ist. Dies ermöglicht ein Verfolgen der an der Strahlenschutzvorrichtung gemessenen Strahlungsbelastung in Echtzeit.

Gemäß der vorliegenden Erfindung ist die Messvorrichtung mit einem (radio freqency identification) RFID-Chip gekoppelt oder selbst als RFID-Chip ausgebildet, der einen Speicher zum Speichern von Daten zur erfassten Strahlung sowie eine Schnittstelle zur drahtlosen Kommunikation mit einer äußeren Sende- und Empfangseinheit, einer Datenverarbeitungseinheit und/oder einer Bilderzeugungseinheit umfasst. Der RFID-Chip bewerkstelligt somit die Speicherung der Daten zur erfassten Strahlung und deren Übermittlung an eine äußere Sende- und Empfangseinheit. Die Kopplung der Messvorrichtung mit einem RFID-Chip ergibt eine besonders vorteilhafte Kombination von Mess- und Aufzeichnungsvorrichtung und damit deren platzsparende Integration in der Strahlenschutzvorrichtung.

Die Sensitivität der Messvorrichtung richtet sich vor allem nach der Strahlungsart, die durch die Strahlenschutzvorrichtung abgeschirmt werden soll. Die Messvorrichtung kann beispielsweise Alpha-Strahlung, Beta-Strahlung, oder Gamma-Strahlung, insbesondere Röntgenstrahlung detektieren, die beispielsweise von einer Röntgen-Strahlungsquelle eines medizinischen oder nicht-medizinischen Untersuchungssystems abgestrahlt wird.

Gemäß einer weiteren Ausführungsform der vorliegenden Erfindung ist die Schutzvorrichtung als Strahlenschutzschürze ausgebildet, die eine aus flexiblem Material gefertigte für die schädliche Strahlung weitgehend undurchlässige Schutzschicht umfasst. Diese Schutzschicht ist zumindest teilweise mit einer äußeren Umschicht umgeben ist, die für die schädliche Strahlung weitgehend durchlässig ist. Dabei ist die Messvorrichtung als Dosimeter ausgebildet, das zwischen der Schutzschicht und der äußeren Umschicht angeordnet ist. Die Integration der Messvorrichtung in der Schutzvorrichtung wird somit erreicht durch die Anordnung der Messvorrichtung bzw. des Dosimeters zwischen der Schutzschicht und der äußeren Umschicht der Strahlenschutzschürze.

Es sind aber auch andere Positionen der Messvorrichtung bzw. des Dosimeters in der Strahlenschutzvorrichtung möglich, ohne den prinzipiellen Gedanken der Integration der vorliegenden Erfindung zu verlassen, der darin besteht, dass zumindest eine Messvorrichtung oder Dosimeter im Material oder Schichtaufbau der Strahlenschutzvorrichtung untergebracht bzw. darin integriert ist.

Vorteilhafterweise ist dazu in der Strahlenschutzschürze eine Tasche oder Aufnahmefach zur Unterbringung vorgesehen, in die der RFID-Chip, die Messvorrichtung, das Dosimeter und/oder die Mess- und Aufzeichnungsvorrichtung einsteckbar ist. Eine solche Tasche kann beispielsweise durch eine umlaufende Naht in der der äußeren Umschicht der Strahlenschutzschürze ausgebildet sein, wobei die umlaufende Naht beispielsweise als Stichnaht mittels eines Nähgarns oder als Schweißnaht ausgebildet sein kann.

Je nach Anwendung und Bedarf kann die Tasche wieder aufmachbar sein, um die Messvorrichtung, den RFID-Chip, das Dosimeter und/oder die Mess- und Aufzeichnungsvorrichtung wieder herausnehmen zu können. Alternativ kann die Tasche verschweißt sein, damit die Tasche nicht geöffnet und die Messvorrichtung, den RFID-Chip, das Dosimeter und/oder die Mess- und Aufzeichnungsvorrichtung nicht aus der Tasche in der Strahlenschutzvorrichtung wieder herausgenommen werden kann. Auf diese Weise ist die Messvorrichtung, der RFID-Chip, das Dosimeter und/oder die Mess- und Aufzeichnungsvorrichtung mit der Strahlenschutzvorrichtung unverlierbar verbunden und eine eindeutige Zuordnung gewährleistet: Ferner können Verwechslungen verhindert werden.

Sofern die Messvorrichtung, den RFID-Chip, das Dosimeter und/oder die Mess- und Aufzeichnungsvorrichtung aus der Tasche in der Strahlenschutzschürze wieder entnehmbar sein soll, kann die Tasche beispielsweise durch einen Reißverschluss oder einen Klettverschluss vorzugsweise an einer seitlichen Naht der Strahlenschutzschürze verschließbar sein. Ein solcher Reißverschluss oder Klettverschluss kann mehrfach verschlossen und wieder geöffnet werden, ohne die Strahlenschutzschürze zu beschädigen. Die Tasche gewährleistet auch eine feste Position der Messvorrichtung, des RFID-Chips, des Dosimeters und/oder der Mess- und Aufzeichnungsvorrichtung im Material bzw. Schichtaufbau der Strahlenschutzschürze.

Gemäß einer weiteren Ausführungsform der vorliegenden Erfindung ist an der Stelle, wo die Messvorrichtung, der RFID-Chip, das Dosimeter und/oder die Mess- und Aufzeichnungsvorrichtung in der Strahlenschutzvorrichtung bzw. Strahlenschutzschürze untergebracht ist, ein für die schädliche Strahlung weitgehend durchlässiges und transparentes Material in der aufliegenden Oberschicht oder Umschicht vorgesehen, so dass die integrierte Messvorrichtung, der RFID-Chip, das Dosimeter und/oder die Mess- und Aufzeichnungsvorrichtung von außen sichtbar ist. So kann durch einfache Sichtkontrolle festgestellt werden, ob die Strahlenschutzvorrichtung bzw. Strahlenschutzschürze mit einer Messvorrichtung, einem RFID-Chip, Dosimeter und/oder einer Mess- und Aufzeichnungsvorrichtung bestückt ist.

Diese äußere Umschicht ist vorzugsweise aus gummiertem, abwaschbarem Material gefertigt, das gegenüber ätzenden Stoffen zumindest zeitweise resistent ist. Ferner kann zum besseren Tragekomfort der Strahlenschutzschürze die äußere Unterschicht oder Umschicht insbesondere an Körperkontaktstellen aus einem hautverträglichen Material gefertigt oder damit bedeckt sein.

Die vorliegende Erfindung löst die oben genannte Ausgabe ferner durch ein Verfahren zum Herstellen einer Vorrichtung zum Schutz vor schädlicher Strahlung, wobei in der Vorrichtung mindestens eine Messvorrichtung zum Detektieren der schädlichen Strahlung integriert wird. Die mindestens eine Messvorrichtung wird auf einer der Einfallsrichtung der Strahlung abgewandten Seite der Schutzschicht angeordnet und ist mit einem RFID-Chip gekoppelt oder selbst als RFID-Chip ausgebildet, der einen Speicher zum Speichern von Daten zur detektierten Strahlung sowie eine Schnittstelle zur drahtgebundenen oder drahtlosen Kommunikation mit einer äußeren Sende- und Empfangseinheit umfasst.

Weitere Einzelheiten, bevorzugte Ausführungsformen und Vorteile der vorliegenden Erfindung ergeben sich aus der nachfolgenden Beschreibung unter Bezugnahme auf die beigefügten Zeichnungen. Es zeigen:
Figur 1 die schematische Darstellung der Vorderseite und Rückseite einer Strahlenschutzschürze ;
Figur 2 die schematische Darstellung der Vorderseite und Rückseite einer weiteren Strahlenschutzschürze ;
Figur 3 eine Detailansicht der Strahlenschutzschürze von Fig. 1 oder Fig. 2 mit einer schematischen Darstellung des Schichtaufbaus ;
Figur 4 die schematische Darstellung der Vorderseite und Rückseite einer Strahlenschutzschürze ; und
Figur 5 eine Detailansicht der Strahlenschutzschürze von Fig. 4 mit einer schematischen Darstellung des Schichtaufbaus .

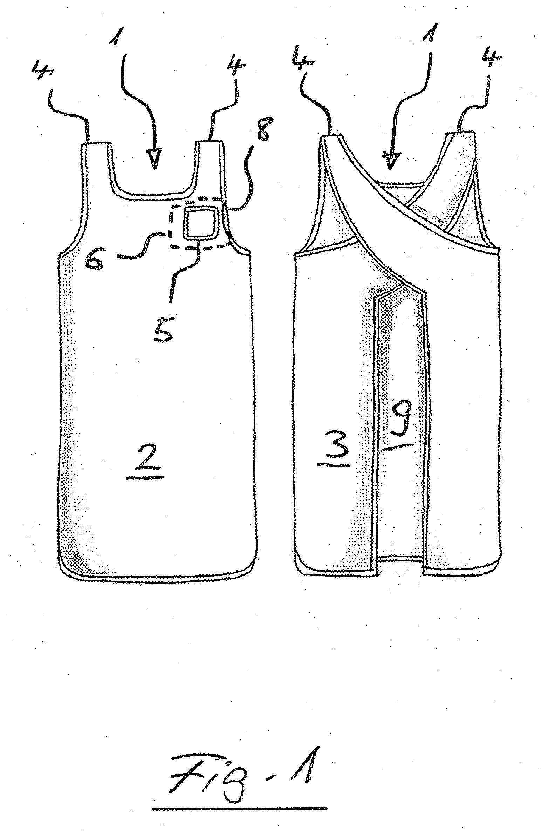

Fig. 1 zeigt jeweils die Vorderseite 2 und Rückseite 3 einer als Strahlenschutzschürze 1 ausgebildeten Strahlenschutzvorrichtung. Bei der in Fig. 1 gezeigten Strahlenschutzvorrichtung handelt es sich um eine tragbare Strahlenschutzschürze 1, die zwei Schulterträger 4 und auf ihrer Rückseite 3 eine senkrechte Öffnung aufweist, wodurch die Innenseite 9 der Strahlenschutzschürze 1 teilweise sichtbar ist. Die Strahlenschutzschürze 1 dient der Abschirmung des Oberkörpers einer Person und enthält Material zur Abschirmung vor der schädlichen Strahlung. Die Strahlenschutzschürze 1 enthält beispielsweise Blei oder bleiähnliches Material, das Röntgenstrahlen abschirmt und ist im medizinischen Bereich beim Umgang mit Röntgenstrahlungsquellen verwendbar.

In der Strahlenschutzschürze 1 ist eine Messvorrichtung 5 integriert, welche die schädliche Strahlung detektiert bzw. erfasst. Dazu ist an der Vorderseite 2 der Strahlenschutzschürze 1 am Ansatz des linken Schulterträgers 4 mit gestrichelter Linie eine Tasche 6 im Material bzw. Schichtaufbau der Strahlenschutzschürze 1 angedeutet, in der die Messvorrichtung 5 untergebracht ist. Die Tasche 6 ist über eine Öffnung 8 in der seitlichen Naht der Strahlenschutzschürze 1 zugänglich. Die Messvorrichtung 5 kann durch diese Öffnung 8 in der seitlichen Naht der Strahlenschutzschürze 1 in die Tasche 6 eingesteckt und so im Material bzw. Schichtaufbau der Strahlenschutzschürze 1 positioniert werden.

Sofern die Messvorrichtung 5 auf der Vorderseite 2 der Strahlenschutzschürze 1 oberhalb einer abschirmenden Materialschicht angeordnet ist und die Strahlung von vorne auf die Strahlenschutzschürze 1 gerichtet ist, misst die Messvorrichtung 5 die auf die Vorderseite 2 der Strahlenschutzschürze 1 auftreffende Strahlung bevor sie von der abschirmenden Materialschicht absorbiert wird. Auf diese Weise kann festgestellt werden, ob die die Strahlenschutzschürze 1 tragende Person einer Strahlung ausgesetzt war und mit welcher Strahlendosis.

Sofern die Messvorrichtung 5 unterhalb der abschirmenden Materialschicht der Strahlenschutzschürze 1 angeordnet ist, misst die Messvorrichtung 5 eine möglicherweise die abschirmende Materialschicht durchdringende Strahlung oder eine vom Körper der die Strahlenschutzschürze 1 tragenden Person selbst abgegeben Strahlung. Auf diese Weise kann festgestellt werden, ob die abschirmende Materialschicht der Strahlenschutzschürze 1 eine ausreichende Wirkung hat oder ob die Person einer Strahlung ausgesetzt war und mit welcher Strahlendosis.

Die umlaufende Naht der Tasche 6 kann beispielsweise als Stichnaht oder als Schweißnaht ausgebildet sein. Die seitliche Öffnung 8 der Tasche 6 ist beispielsweise durch einen Reißverschluss oder einen Klettverschluss verschließbar und somit wieder aufmachbar, so dass die Messvorrichtung 5 über die Öffnung 8 aus der Tasche 6 wieder entnommen werden kann. Alternativ kann die Öffnung 8 der Tasche 6 verschweißt sein, damit die Tasche 6 nicht geöffnet werden kann, so dass die Messvorrichtung 5 nicht aus der Tasche 6 entnommen werden kann, ohne die Tasche 6 zu zerstören. Damit wäre die Messvorrichtung 5 mit der Strahlenschutzschürze 1 unverlierbar verbunden.

Die Messvorrichtung 5 kann auch mit einer Aufzeichnungsvorrichtung ausgestattet sein, welche die von der Messvorrichtung 5 erfassten Messwerte zur detektierten Strahlung speichert. Diese Aufzeichnungsvorrichtung ist vorzugsweise ebenfalls in der Tasche 6 untergebracht und damit in der Strahlenschutzschürze 1 integriert. Anstelle der Messvorrichtung 5 kann auch eine kombinierte Mess- und Aufzeichnungsvorrichtung eingesetzt werden oder ein Dosimeter, das sowohl die Dosis der detektierten Strahlung erfasst als auch die erfassten Messwerte der Strahlungsdosis speichert. Ferner kann die Messvorrichtung 5 mit einem RFID-Chip gekoppelt sein, der einen Speicher zum Speichern von Daten zur erfassten Strahlung sowie eine Schnittstelle zur drahtlosen Kommunikation mit einer äußeren Sende- und Empfangseinheit (nicht gezeigt) bereit stellt.

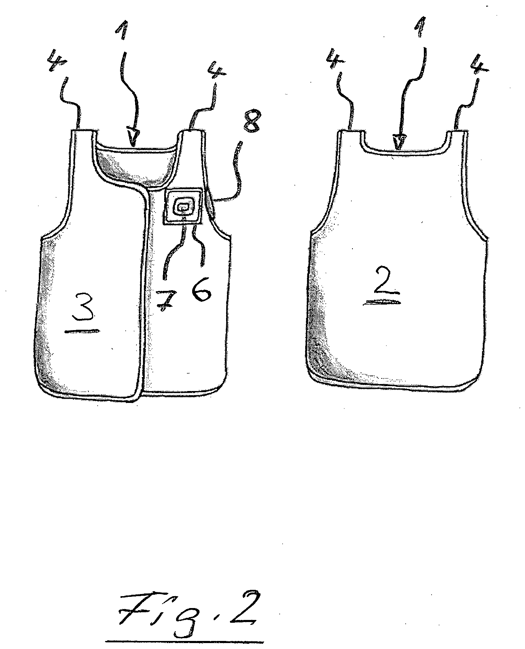

Fig. 2 zeigt eine die schematische Ansicht auf die Vorderseite 2 und Rückseite 3 einer tragbaren Strahlenschutzschürze 1. Diese Strahlenschutzschürze 1 umfasst wiederum zwei Schulterträger 4 und ist auf ihrer Rückseite 3 mit einer Überlappung verschließbar. Bei dieser Ausführungsform in der Strahlenschutzschürze 1 ist auf ihrer Rückseite 3 eine kombinierte Mess- und Aufzeichnungsvorrichtung 7 integriert, welche die schädliche Strahlung detektiert und die erfassten Messwerte bzw. die detektierten Strahlungsdosen speichert.

Dazu ist in Fig. 2 auf der Rückseite 3 der Strahlenschutzschürze 1 am Ansatz des rechten Schulterträgers 4 mit doppelter Linie eine Tasche 6 im Material bzw. Schichtaufbau der Strahlenschutzschürze 1 angedeutet, in der die Mess- und Aufzeichnungsvorrichtung 7 untergebracht ist. Die Tasche 6 ist wiederum über eine Öffnung 8 in der seitlichen Naht der Strahlenschutzschürze 1 zugänglich. Die Mess- und Aufzeichnungsvorrichtung 7 kann durch diese Öffnung 8 in der seitlichen Naht der Strahlenschutzschürze 1 in die Tasche 6 eingesteckt und so im Material bzw. Schichtaufbau der Strahlenschutzschürze 1 positioniert werden. Wenn die Mess- und Aufzeichnungsvorrichtung 7 auf der Rückseite 3 der Strahlenschutzschürze 1 oberhalb der abschirmenden Materialschicht angeordnet ist und die Strahlung von hinten auf die Strahlenschutzschürze 1 gerichtet ist, misst die Messvorrichtung 5 die auf die Rückseite 3 der Strahlenschutzschürze 1 auftreffende Strahlung bevor sie von der abschirmenden Materialschicht absorbiert wird. Auf diese Weise kann festgestellt werden, ob die die Strahlenschutzschürze 1 tragende Person einer Strahlung ausgesetzt war und mit welcher Strahlendosis.

Sofern die Mess- und Aufzeichnungsvorrichtung 7 unterhalb der abschirmenden Materialschicht der Strahlenschutzschürze 1 angeordnet ist, misst sie eine möglicherweise die abschirmende Materialschicht durchdringende Strahlung oder eine vom Körper der die Strahlenschutzschürze 1 tragenden Person selbst abgegeben Strahlung. Auf diese Weise kann festgestellt werden, ob die die abschirmende Materialschicht der Strahlenschutzschürze 1 eine ausreichende Wirkung hat oder ob die Person einer Strahlung ausgesetzt war und mit welcher Strahlendosis.

Es können auch die in Fig. 1 und in Fig. 2 dargestellten Ausführungsformen der Strahlenschutzschürzen 1 miteinander kombiniert werden, so dass sowohl auf der Vorderseite 2 als auch auf der Rückseite 3 der Strahlenschutzschürze 1 eine Messvorrichtung 5 oder eine Mess- und Aufzeichnungsvorrichtung 7 integriert ist, welche die schädliche Strahlung detektiert. Dabei können mehrere Messvorrichtung 5 oder Mess- und Aufzeichnungsvorrichtungen 7 bezüglich der Einfallsrichtung der Strahlung sowohl oberhalb als auch unterhalb der abschirmenden Materialschicht der Strahlenschutzschürze 1 angeordnet sein.

Wie bei der in Fig. 1 dargestellten Strahlenschutzschürze 1 kann auch bei der in Fig. 2 dargestellten Strahlenschutzschürze die Mess- und Aufzeichnungsvorrichtung 7 mit einem RFID-Chip gekoppelt sein oder selbst als RFID-Chip ausgebildet sein, der einen Speicher zum Speichern von Daten zur erfassten Strahlung sowie eine Schnittstelle zur drahtlosen Kommunikation mit einer äußeren Sende- und Empfangseinheit umfasst.

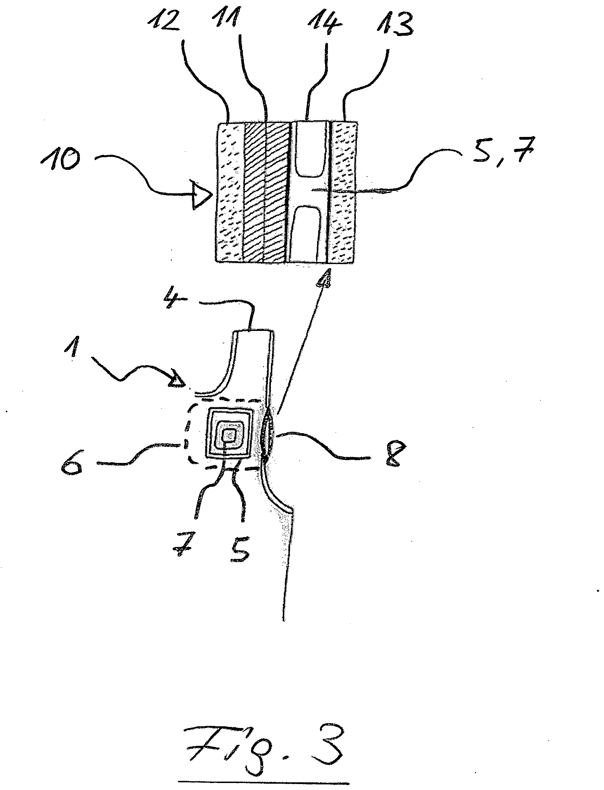

Der untere Teil von Fig. 3 zeigt eine Detailansicht der in Fig. 1 oder Fig. 2 dargestellten Strahlenschutzschürze 1 und der obere Teil von Fig. 3 zeigt eine schematische Darstellung des Schichtaufbaus der Strahlenschutzschürze 1 _{.}

In der Detailansicht im unteren Teil von Fig. 3 ist der Bereich am Schulterträger 4 einer der in Fig. 1 oder Fig. 2 dargestellten Strahlenschutzschürze 1 vergrößert gezeigt. In der Strahlenschutzschürze 1 ist eine Messvorrichtung 5 oder eine Mess- und Aufzeichnungsvorrichtung 7 integriert, welche die schädliche Strahlung detektiert bzw. erfasst. Dabei kann die Messvorrichtung 5 oder Mess- und Aufzeichnungsvorrichtung 7 als Dosimeter mit einem RFID-Chip ausgebildet und/oder damit gekoppelt sein.

Im Bereich unterhalb des Schulterträgers 4, der auf der Vorderseite 2 oder auf der Rückseite 3 der Strahlenschutzschürze 1 liegen kann, ist mit gestrichelter Linie eine Tasche 6 im Material der Strahlenschutzschürze 1 angedeutet, in der die Messvorrichtung 5, die Mess- und Aufzeichnungsvorrichtung 7, der RFID-Chip oder das Dosimeter untergebracht ist. Über die Öffnung 8 in der seitlichen Naht am Träger 4 der Strahlenschutzschürze 1 ist die Tasche 6 zugänglich. Die Messvorrichtung 5, die Mess- und Aufzeichnungsvorrichtung 7, der RFID-Chip oder das Dosimeter kann durch diese Öffnung 8 in der seitlichen Naht der Strahlenschutzschürze 1 in die Tasche 6 eingesteckt und so im Material bzw. im Schichtaufbau 10 der Strahlenschutzschürze 1 positioniert werden.

Dieser Schichtaufbau 10 der Strahlenschutzschürze 1 ist im oberen Teil Fig. 3 in einem Querschnitt schematisch gezeigt. Den Kern der Strahlenschutzschürze 1 stellt die Schutzschicht 11 dar, die ein Strahlung absorbierendes bzw. abschirmendes Material umfasst. Die Schutzschicht 11 ist von einer Umschicht umgeben, die auf der Innenseite 9 der Strahlenschutzschürze 1 eine Innenschicht 12 aufweist und auf der Außenseite 2, 3 der Strahlenschutzschürze 1 eine Außenschicht 13 umfasst. Zwischen der Schutzschicht 11 und der Außenschicht 13 befindet sich eine Integralschicht 14, in der die Messvorrichtung 5, die Mess- und Aufzeichnungsvorrichtung 7, der RFID-Chip oder das Dosimeter angeordnet ist.

Mit Bezug auf die in Fig. 1, oder Fig. 2 dargestellten Strahlenschutzschürzen 1 befindet sich in der Integralschicht 14 die Tasche 6, in der die Messvorrichtung 5, die Mess- und Aufzeichnungsvorrichtung 7, der RFID-Chip oder das Dosimeter Untergebracht ist. Bei dem in Fig. 3 dargestellten Schichtaufbau misst die in der Integralschicht 14 untergebrachte Messvorrichtung 5 bzw. Mess- und Aufzeichnungsvorrichtung 7 die durch die Außenschicht 13 einfallende Strahlung bevor sie die Schutzschicht 11 erreicht.

Diese Außenschicht 13 ist vorzugsweise aus gummiertem, abwaschbarem Material gefertigt, das gegenüber ätzenden Stoffen zumindest zeitweise resistent ist, während die Innenschicht 12 insbesondere an Körperkontaktstellen aus einem hautverträglichen Material gefertigt oder damit bedeckt ist.

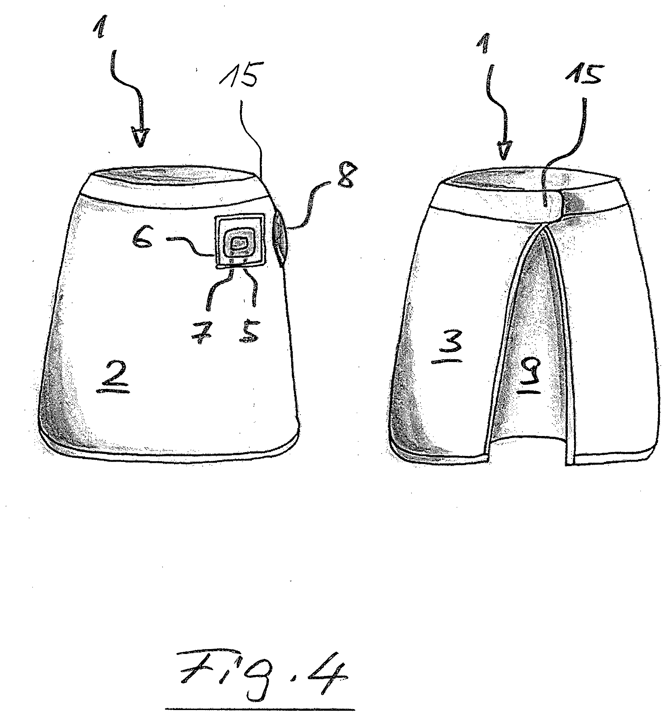

Fig. 4 zeigt die schematische Darstellung einer Strahlenschutzvorrichtung, wobei jeweils die Vorderseite 2 und Rückseite 3 der als Strahlenschutzschürze 1 ausgebildeten Strahlenschutzvorrichtung gezeigt ist. Bei dieser Strahlenschutzvorrichtung handelt es sich um einen Strahlenschutzrock 1, der am oberen Rand einen Bund 15 und auf der Rückseite eine senkrechte Öffnung aufweist, wodurch die Innenseite 9 der Strahlenschutzschürze 1 teilweise sichtbar ist. Die Strahlenschutzschürze 1 dient insbesondere der Abschirmung des Unterkörpers einer Person und enthält entsprechendes Material zur Abschirmung vor der schädlichen Strahlung.

In der Strahlenschutzschürze 1 ist eine Messvorrichtung 5 und eine mit der Messvorrichtung 5 gekoppelte Aufzeichnungsvorrichtung 7 integriert, welche die schädliche Strahlung detektiert und die gemessenen Strahlungswerte speichert. An der Vorderseite 2 der Strahlenschutzschürze 1 ist eine Tasche 6 im Material der Strahlenschutzschürze 1vorgesehen, in der die Messvorrichtung 5 und die Aufzeichnungsvorrichtung 7 untergebracht sind. Die Tasche 6 ist über eine Öffnung 8 in der seitlichen Naht der Strahlenschutzschürze 1 zugänglich. Die Messvorrichtung 5 und die Aufzeichnungsvorrichtung 7 können jeweils durch diese Öffnung 8 in der seitlichen Naht der Strahlenschutzschürze 1 in die Tasche 6 eingesteckt und so im Material bzw. Schichtaufbau der Strahlenschutzschürze 1 positioniert und damit darin integriert werden.

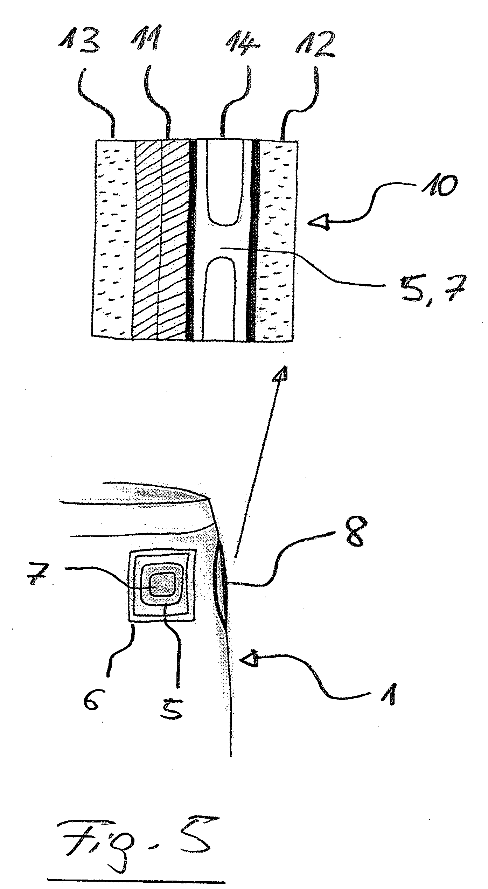

Im unteren Teil von Fig. 5 ist eine Detailansicht des in Fig. 4 dargestellten Strahlenschutzrocks 1 vergrößert gezeigt. Im oberen Teil von Fig. 5 ist der Schichtaufbau der in Fig. 4 dargestellten Ausführungsform des Strahlenschutzrocks 1 in einem Querschnitt schematisch gezeigt. Im diesem Schichtaufbau des Strahlenschutzrocks 1 ist eine Messvorrichtung 5 oder eine Mess- und Aufzeichnungsvorrichtung 7 integriert, welche die schädliche Strahlung detektiert bzw. erfasst. Dabei kann die Messvorrichtung 5 oder Mess- und Aufzeichnungsvorrichtung 7 als Dosimeter oder als RFID-Chip ausgebildet und/oder damit gekoppelt sein.

Die Messvorrichtung 5, die Mess- und Aufzeichnungsvorrichtung 7, der RFID-Chip oder das Dosimeter sind in einer Tasche 6 untergebracht, die über eine Öffnung 8 in der seitlichen Naht des Strahlenschutzrocks 1 zugänglich ist. Durch diese Öffnung 8 in der seitlichen Naht der Strahlenschutzschürze 1 in die Tasche 6 kann die Messvorrichtung 5, die Mess- und Aufzeichnungsvorrichtung 7, der RFID-Chip oder das Dosimeter eingesteckt und so im Material bzw. im Schichtaufbau 10 der Strahlenschutzschürze 1 positioniert werden.

Die Strahlenschutzschürze 1 umfasst eine Schutzschicht 11, die ein Strahlung absorbierendes bzw. abschirmendes Material enthält. Die Schutzschicht 11 ist von einer Umschicht umgeben, die auf der Innenseite 9 der Strahlenschutzschürze 1 eine Innenschicht 12 aufweist und auf der Außenseite 2, 3 der Strahlenschutzschürze 1 eine Außenschicht 13 aufweist.

Bei der in Fig. 5 dargestellten Strahlenschutzschürze befindet sich zwischen der Schutzschicht 11 und der Innenschicht 12 eine Integralschicht 14, in der die Messvorrichtung 5, die Mess- und Aufzeichnungsvorrichtung 7, der RFID-Chip oder das Dosimeter angeordnet ist. Mit diesem Schichtaufbau misst die in der Integralschicht 14 untergebrachte Messvorrichtung 5 bzw. Mess- und Aufzeichnungsvorrichtung 7 die durch die Innenschicht 12 einfallende Strahlung. Folglich wird bei diesem Aufbau eine möglicherweise die abschirmende Materialschicht 11 durchdringende Strahlung oder eine vom Körper der den Strahlenschutzrock 1 tragenden Person selbst abgegeben Strahlung gemessen. Auf diese Weise kann festgestellt werden, ob die die abschirmende Materialschicht der Strahlenschutzschürze 1 eine ausreichende Wirkung hat oder ob die Person einer Strahlung ausgesetzt war und mit welcher Strahlendosis.

Es können auch die in Fig. 3 und in Fig. 5 dargestellten Schichtaufbauten der erfindungsgemäßen Strahlenschutzschürzen 1 miteinander kombiniert werden, so dass eine Messvorrichtung 5 oder eine Mess- und Aufzeichnungsvorrichtung 7 bezüglich der Einfallsrichtung der Strahlung sowohl oberhalb der abschirmenden Materialschicht der Strahlenschutzschürze 1 angeordnet ist als auch eine Messvorrichtung 5 oder eine Mess- und Aufzeichnungsvorrichtung 7 bezüglich der Einfallsrichtung der Strahlung unterhalb der abschirmenden Materialschicht der Strahlenschutzschürze 1 angeordnet ist.

Während die vorliegende Beschreibung exemplarische Ausführungsformen der Erfindung im Detail angibt, sind die in den beigefügten Zeichnungen dargestellten Ausführungsformen lediglich illustrativ zu verstehen und nicht einschränkend für den Schutzbereich der Erfindung auszulegen. Es wird deshalb darauf hingewiesen, dass verschiedene Modifikationen an den beschriebenen, dargestellten oder anderen Ausführungsformen der Erfindung vorgenommen werden können, ohne von dem durch die beigefügten Ansprüche definierten Schutzumfang abzuweichen.

### Liste der Bezugszeichen

- 1: Strahlenschutzschürze
- 2: Vorderseite der Strahlenschutzschürze
- 3: Rückseite der Strahlenschutzschürze
- 4: Schulterträger der Strahlenschutzschürze
- 5: Messvorrichtung
- 6: Tasche in der Strahlenschutzschürze
- 7: Mess- und Aufzeichnungsvorrichtung
- 8: Öffnung zur Tasche 6
- 9: Innenseite der Strahlenschutzschürze
- 10: Schichtaufbau der Strahlenschutzschürze
- 11: Schutzschicht
- 12: Innenschicht
- 13: Außenschicht
- 14: Integralschicht
- 15: Bund der Strahlenschutzschürze

## Patentansprüche

1. Vorrichtung zum Schutz vor schädlicher Strahlung, die eine Schutzschicht (11) zum Schutz vor der schädlichen Strahlung umfasst, wobei in der Schutzvorrichtung (1) mindestens eine Messvorrichtung (5) zum Detektieren der schädlichen Strahlung integriert ist, wobei die mindestens eine Messvorrichtung (5) auf einer der Einfallsrichtung der Strahlung abgewandten Seite der Schutzschicht (11) angeordnet ist;
**dadurch gekennzeichnet dass** die Messvorrichtung (5) mit einem RFID-Chip (7) gekoppelt oder selbst als RFID Chip ausgebildet ist, der einen Speicher zum Speichern von Daten zur detektierten Strahlung sowie eine Schnittstelle zur drahtlosen Kommunikation mit einer äußeren Sende- und Empfangseinheit umfasst.

2. Vorrichtung nach Anspruch 1, wobei eine weitere Messvorrichtung (5) auf einer der Einfallsrichtung der Strahlung zugewandten Seite der Schutzschicht (11) angeordnet ist.

3. Vorrichtung nach einem der vorangehenden Ansprüche, wobei Mittel zur Erfassung der räumlichen Position der mindestens einen Messvorrichtung (5) vorgesehen sind.

4. Vorrichtung nach einem der vorangehenden Ansprüche, wobei die Schnittstelle zur drahtgebundenen oder drahtlosen Kommunikation eine Bandbreite aufweist, die eine verzögerungsfreie Übertragung der Daten zur detektierten Strahlung in Echtzeit ermöglicht.

5. Vorrichtung nach einem der vorangehenden Ansprüche, wobei die Schnittstelle weiterhin ausgebildet ist zur drahtgebundenen oder drahtlosen Kommunikation mit einer Datenverarbeitungseinheit und/oder Bilderzeugungseinheit ,

6. Vorrichtung nach einem der vorangehenden Ansprüche, wobei die Daten zur detektierten Strahlung Informationen zur Dosis, Zeitpunkt, Zeitspanne, Intensität, Strahlungsart, Strahlungsrate, Gesamtmenge der erfassten Strahlung, räumlichen Position der mindestens einen Messvorrichtung (5) und/oder dem zeitlichen Verlauf der genannten Observablen umfassen.

7. Vorrichtung nach einem der vorangehenden Ansprüche, wobei die mindestens eine Messvorrichtung (5) insbesondere Alpha-Strahlung, Beta-Strahlung, oder Gamma-Strahlung, insbesondere Röntgenstrahlung detektiert.

8. Vorrichtung nach einem der vorangehenden Ansprüche, wobei die Schutzvorrichtung (1) als Strahlenschutzschürze ausgebildet ist, die eine aus flexiblem Material gefertigte für die schädliche Strahlung weitgehend undurchlässige Schutzschicht (11) umfasst, die zumindest teilweise mit einer äußeren Umschicht (12, 13) umgeben ist, die für die schädliche Strahlung weitgehend durchlässig ist, und die mindestens eine Messvorrichtung (5) als Dosimeter ausgebildet ist, das zwischen der Schutzschicht (11) und der äußeren Umschicht (12, 13) angeordnet ist.

9. Vorrichtung nach dem vorangehenden Anspruch, wobei in der Strahlenschutzschürze (1) eine Tasche (6) oder Aufnahmefach vorgesehen ist, in die das Dosimeter einsteckbar ist.

10. Vorrichtung nach dem vorangehenden Anspruch, wobei die Tasche (6) durch eine umlaufende Naht in der der äußeren Umschicht (12, 13) der Strahlenschutzschürze (1) ausgebildet ist.

11. Vorrichtung nach einem der Ansprüche 9 oder 10, wobei die Tasche (6) durch einen Reißverschluss oder einen Klettverschluss an einer seitlichen Naht der Strahlenschutzschürze (1) verschließbar ist.

12. Vorrichtung nach einem der Ansprüche 7 bis 11, wobei an der Stelle des Dosimeters ein für die schädliche Strahlung weitgehend durchlässiges und transparentes Material in der Umschicht (12, 13) vorgesehen ist, so dass das integrierte Dosimeter von außen sichtbar ist.

13. Vorrichtung nach einem der Ansprüche 7 bis 12, wobei die äußere Umschicht (12, 13) aus gummiertem, abwaschbarem Material gefertigt ist, das gegenüber ätzenden Stoffen zumindest zeitweise resistent ist.

14. Vorrichtung nach einem der Ansprüche 7 bis 13, wobei die äußere Umschicht (12, 13) zum Tragekomfort der Strahlenschutzschürze an Körperkontaktstellen (9) aus einem hautverträglichen Material gefertigt oder damit bedeckt ist.

15. Verfahren zum Herstellen einer Vorrichtung zum Schutz vor schädlicher Strahlung, wobei die Vorrichtung eine Schutzschicht (11) zum Schutz vor der schädlichen Strahlung umfasst, wobei in der Schutzvorrichtung (1) mindestens eine Messvorrichtung (5) zum Detektieren der schädlichen Strahlung integriert wird, und wobei die mindestens eine Messvorrichtung (5) auf einer der Einfallsrichtung der Strahlung abgewandten Seite der Schutzschicht (11) angeordnet wird; **dadurch gekennzeichnet dass** die Messvorrichtung (5) mit einem RFID-Chip (7) gekoppelt oder selbst als RFID-Chip ausgebildet ist, der einen Speicher zum Speichern von Daten zur erfassten Strahlung sowie eine Schnittstelle zur drahtlosen Kommunikation mit einer äußeren Sende- und Empfangseinheit umfasst.

## Claims

1. A device for protection from harmful radiation, comprising a protective layer (11) for protection from the harmful radiation, wherein at least one measuring device (5) for detecting the harmful radiation is integrated in the protection device (1), wherein the at least one measuring device (5) is arranged at a side of the protective layer (11) facing away from the direction of arrival of the radiation;
**characterized in that**
the measuring device (5) is coupled with an RFID chip (7) or is itself designed as an RFID chip comprising a storage means for storing data concerning the radiation detected as well as an interface for the wireless communication with an external sending and receiving unit.

2. The device according to claim 1, wherein a further measuring device (5) is arranged at a side of the protective layer (11) facing the direction of arrival of the radiation.

3. The device according to any of the preceding claims, wherein means for detecting the spatial position of the at least one measuring device (5) are provided.

4. The device according to any of the preceding claims, wherein the interface for the wired or wireless communication has a bandwidth enabling a delay-free real-time transmission of the data concerning the radiation detected.

5. The device according to any of the preceding claims, wherein the interface is further designed for the wired or wireless communication with a data processing unit and/or image generating unit.

6. The device according to any of the preceding claims, wherein the data concerning the radiation detected comprise information about the dose, point in time, period, intensity, type of radiation, radiation rate, total amount of radiation detected, spatial position of the at least one measuring device (5) and/or the chronological sequence of the observables mentioned.

7. The device according to any of the preceding claims, wherein the at least one measuring device (5) detects in particular alpha radiation, beta radiation, or gamma radiation, in particular X-radiation.

8. The device according to any of the preceding claims, wherein the protection device (1) is designed as a protective apron comprising a protective layer (11) manufactured of a flexible material and being largely impermeable for the harmful radiation, which is at least partially surrounded with an external surrounding layer (12, 13) that is largely permeable for the harmful radiation, and wherein the at least one measuring device (5) is designed as a dosimeter arranged between the protective layer (11) and the external surrounding layer (12, 13).

9. The device according to the preceding claim wherein a pocket (6) or receptacle is provided in the protective apron (1) into which the dosimeter is insertable.

10. The device according to the preceding claim, wherein the pocket (6) is formed by a circumferential seam in the external surrounding layer (12, 13) of the protective apron (1).

11. The device according to any of claims 9 or 10, wherein the pocket (6) is closable by a zipper or a hook and loop fastener at a lateral seam of the protective apron (1).

12. The device according to any of claims 7 to 11, wherein, at the place of the dosimeter, a transparent material which is largely permeable for the harmful radiation is provided in the surrounding layer (12, 13), so that the integrated dosimeter is visible from outside.

13. The device according to any of claims 7 to 12, wherein the external surrounding layer (12, 13) is manufactured of a rubberized, washable material which is at least temporarily resistant to etching substances.

14. The device according to any of claims 7 to 13, wherein the external surrounding layer (12, 13) is manufactured of or covered with a skin-friendly material for the sake of wearing comfort of the protective apron at places making body contact (9).

15. A method for manufacturing a device for protection from harmful radiation , wherein the device comprises a protective layer (11) for protection from the harmful radiation, wherein at least one measuring device (5) for detecting the harmful radiation is integrated in the protection device (1), and wherein the at least one measuring device (5) is arranged at a side of the protective layer (11) facing away from the direction of arrival of the radiation;
**characterized in that**
the measuring device (5) is coupled with an RFID chip (7) or is itself designed as an RFID chip comprising a storage means for storing data concerning the radiation detected as well as an interface for the wireless communication with an external sending and receiving unit.

## Revendications

1. Dispositif de protection contre un rayonnement nocif, qui comprend une couche de protection (11) pour la protection contre le rayonnement nocif, dans lequel au moins un dispositif de mesure (5) pour la détection du rayonnement nocif est intégré dans le dispositif de protection (1),
dans lequel ledit au moins un dispositif de mesure (5) est disposé sur un côté de la couche de protection (11) opposé à la direction d'incidence du rayonnement ;
**caractérisé en ce que**
le dispositif de mesure (5) est couplé à une puce RFID (7) ou est luimême conçu comme une puce RFID qui comprend une mémoire pour stocker des données relatives au rayonnement détecté ainsi qu'une interface pour la communication sans fil avec une unité d'émission et de réception extérieure.

2. Dispositif selon la revendication 1, dans lequel un autre dispositif de mesure (5) est disposé sur un côté de la couche de protection (11) tourné vers la direction d'incidence du rayonnement.

3. Dispositif selon l'une des revendications précédentes, dans lequel des moyens sont prévus pour détecter la position spatiale dudit au moins un dispositif de mesure (5).

4. Dispositif selon l'une des revendications précédentes, dans lequel l'interface pour la communication filaire ou sans fil a une largeur de bande permettant une transmission sans retard des données relatives au rayonnement détecté en temps réel.

5. Dispositif selon l'une des revendications précédentes, dans lequel l'interface est en outre conçue pour la communication filaire ou sans fil avec une unité de traitement de données et/ou une unité de génération d'images.

6. Dispositif selon l'une des revendications précédentes, dans lequel les données relatives au rayonnement détecté comprennent des informations sur la dose, l'instant, la durée, l'intensité, le type de rayonnement, le taux de rayonnement, la quantité totale du rayonnement détecté, la position spatiale dudit au moins un dispositif de mesure (5) et/ou l'évolution dans le temps desdites observables.

7. Dispositif selon l'une des revendications précédentes, dans lequel ledit au moins un dispositif de mesure (5) détecte en particulier un rayonnement alpha, un rayonnement bêta ou un rayonnement gamma, en particulier un rayonnement X.

8. Dispositif selon l'une des revendications précédentes, dans lequel le dispositif de protection (1) est réalisé sous la forme d'un tablier de radioprotection qui comprend une couche de protection (11) fabriquée dans un matériau flexible et largement imperméable au rayonnement nocif, qui est entourée au moins partiellement d'une couche d'enveloppe extérieure (12, 13) qui est largement perméable au rayonnement nocif, et ledit au moins un dispositif de mesure (5) est réalisé sous la forme d'un dosimètre qui est disposé entre la couche de protection (11) et la couche d'enveloppe extérieure (12, 13).

9. Dispositif selon la revendication précédente, dans lequel une poche (6) ou un compartiment de réception, dans laquelle ou lequel le dosimètre peut être inséré, est prévu(e) dans le tablier de radioprotection (1).

10. Dispositif selon la revendication précédente, dans lequel la poche (6) est formée par une couture périphérique dans la couche d'enveloppe extérieure (12, 13) du tablier de radioprotection (1).

11. Dispositif selon l'une des revendications 9 ou 10, dans lequel la poche (6) peut être fermée par une fermeture à glissière ou par une fermeture velcro sur une couture latérale du tablier de radioprotection (1).

12. Dispositif selon l'une des revendications 7 à 11, dans lequel un matériau largement transparent et perméable au rayonnement nocif est prévu dans la couche d'enveloppe (12, 13) à l'emplacement du dosimètre, de sorte que le dosimètre intégré est visible de l'extérieur.

13. Dispositif selon l'une des revendications 7 à 12, dans lequel la couche d'enveloppe extérieure (12, 13) est fabriquée dans un matériau caoutchouté lavable, qui résiste au moins temporairement aux substances corrosives.

14. Dispositif selon l'une des revendications 7 à 13, dans lequel la couche d'enveloppe extérieure (12, 13) est fabriquée dans un matériau compatible avec la peau ou recouverte d'un tel matériau pour le confort de port du tablier de radioprotection aux endroits de contact avec le corps (9).

15. Procédé de fabrication d'un dispositif de protection contre un rayonnement nocif, le dispositif comprenant une couche de protection (11) pour la protection contre le rayonnement nocif, dans lequel au moins un dispositif de mesure (5) pour la détection du rayonnement nocif est intégré dans le dispositif de protection (1), et dans lequel ledit au moins un dispositif de mesure (5) est disposé sur un côté de la couche de protection (11) opposé à la direction d'incidence du rayonnement ;
**caractérisé en ce que**
le dispositif de mesure (5) est couplé à une puce RFID (7) ou est lui-même conçu comme une puce RFID qui comprend une mémoire pour stocker des données relatives au rayonnement détecté ainsi qu'une interface pour la communication sans fil avec une unité d'émission et de réception extérieure.
